# EUROPEAN PATENT SPECIFICATION

(11) **EP 0 653 182 B1**
(45) Date of publication and mention of the grant of the patent: **19.01.2000**
(21) Application number: 94116466.7
(22) Date of filing: 19.10.1994
(51) Int. Cl.: A61B 5/024, H01R 13/62

(54) **Portable measuring apparatus**
Tragbares Messgerät
Appareil de mesure portatif

(30) Priority: 25.10.1993 JP 26645693; 23.08.1994 JP 19860694
(43) Date of publication of application: 17.05.1995
(73) Proprietor: SEIKO EPSON CORPORATION, Shinjuku-ku, Tokyo 163-0811 (JP)
(72) Inventor: Kondo, Yutaka, c/o Seiko Epson Corp., Suwa-shi, Nagano-ken 392 (JP)
(74) Representative: Hoffmann, Eckart, Dipl.-Ing.

(56) References cited:
- EP-A- 0 289 208
- WO-A-91/18550
- CH-A- 591 233
- DE-A- 3 040 524
- GB-A- 2 052 051
- US-A- 4 295 472
- US-A- 4 407 295
- PATENT ABSTRACTS OF JAPAN vol. 7, no. 260 (P-237) 18 November 1983 & JP-A-58 142 289 (TOKYO SHIBAURA DENKI K. K.) 24 August 1983
- PATENT ABSTRACTS OF JAPAN vol. 4, no. 39 (P-004) 28 March 1980 & JP-A-55 010 570 (CITIZEN WATCH CO. LTD.) 25 January 1980

## Description

The present invention relates to a portable measuring apparatus as defined in the precharacterizing portion of claim 1.

US-A-4,407,295 discloses such portable measuring apparatus, in particular a wrist-worn apparatus, in which a sensor for measuring physiological data, such as the pulse rate, or environment data is incorporated in a wrist watch type wrist side measuring device. In this portable apparatus, a sensor for measuring the pulse rate is disposed on a portion of the side portion of a wrist watch type apparatus body, while a connector is disposed on a portion of the side surface of the apparatus body which is remotest from the sensor so that an external measuring device for measuring the heart sound can be detachably connected thereto. Another example of the conventional portable apparatus, disclosed in US-A-4,450,843, is constructed such that a connector of a sensor for measuring the pulse rate is detachably connected to the side portion of a wrist watch type apparatus body. GB-A-2 052 051 discloses a digital watch having a pulse sensor unit detachably mounted via a connector provided on the front surface portion of the watch case.

However, the above-described conventional portable measuring apparatuses are insufficient in terms of the ease with which the apparatus is used or the function thereof. Particularly, in recent years, there is a tendency that the portable apparatus is used during exercise for health care, and hence there has been an increasing demand for a portable measuring system which is capable of general measurements.

The document EP-A-0289 208 discloses an electrical connector apparatus has a connector socket and a connector plug which can cooperate so that electrical contacts of the connector socket engage respective electrical contacts of the plug and the connector socket and the connector plug are kept in engagement with each other by magnetic force, whereby when unintentional tension is applied to a cord coupled to the plug, the cord will not be broken since the connector socket and the connector plug can be easily disengaged from each other.

In view of the aforementioned problems, a primary object of the present invention is to provide a portable measuring apparatus which is easy to use and which is capable of general measurements and which assures firm connection of a connector of an external unit by a magnetic force, and is capable of preventing adverse influence of the magnetic force on an apparatus body.

These objects are achieved with a portable measuring apparatus as claimed.

Preferred embodiments of the invention are subject-matter of the dependent claims.

In the present invention, since a loop of lines of magnetic force is formed in at least either the magnets of the connector of the external unit or the magnetizable members of the wrist side apparatus body, a sufficient magnetic force can be obtained and the connector can be firmly connected even when no magnet is provided in the apparatus body. Further, since no magnet is provided in the apparatus body, problems involving attachment of dust, such as iron sand, or erasure of the data on a magnetic card can be prevented.

Preferred embodiments of the invention will be described in detail below with reference to the drawings, in which:
- Fig. 1: is a plan view of a portable apparatus;
- Fig. 2: is a cross-section taken along the line II - II of Fig. 1;
- Fig. 3: is a cross-section taken along the line III - III of Fig. 1;
- Fig. 4(a): is a plan view illustrating a modification of an operation button shown in Fig. 1;
- Fig. 4(b): is a cross-section taken along the line B - B of Fig. 4 (a);
- Fig. 5: is a flowchart showing the procedures of using the portable apparatus;
- Fig. 6: illustrates how an embodiment of the portable apparatus according to the present invention is mounted on an arm;
- Fig. 7: is a cross-section taken along the line VII - VII of Fig. 6;
- Fig. 8: is a plan view of an acceleration sensor of Fig. 7;
- Fig. 9(a): is a longitudinal cross-sectional view showing how an external measuring device is mounted on a finger;
- Fig. 9(b): is a lateral cross-sectional view of Fig. 9 (a);
- Fig. 10: is a plan view of the portable apparatus;
- Fig. 11: is a side cross-sectional view showing a connector portion when an external measuring device is mounted on a wrist side measuring device;
- Fig. 12: is a plan view illustrating still another embodiment according to the present invention;
- Fig. 13: is a cross-sectional view illustrating a connector portion when an external measuring device is mounted on a wrist side measuring device; and
- Fig. 14: is a cross-section taken along the line VII - VII of Fig. 6 illustrating a modification in which the connector portion shown in Fig. 6 is provided on a front surface of a band;

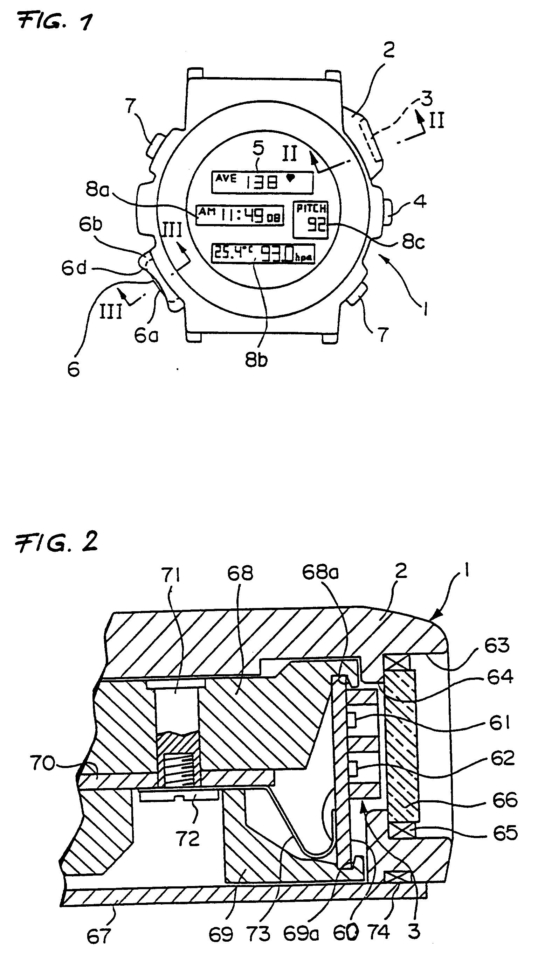

A first embodiment of a portable apparatus will be described below with reference to Figs. 1 through 5. Fig. 1 is a plan view showing a portable apparatus. Referring to Fig. 1, reference numeral 1 denotes an apparatus body. The apparatus body 1 has almost the same shape and size as those of a wrist watch. The apparatus body 1 is attached to an arm using a band which is not shown. The apparatus body 1 has a protruding portion 2 at a side portion thereof. The protruding portion 2 accommodates an infrared radiation sensor (a bio sensor) 3. As shown in Fig. 2, the infrared radiation sensor 3 includes an infrared radiation sensor substrate 60, a light-emitting element 61 and a light-receiving element (a measuring element) 62 which are connected to the substrate 60. The protruding portion 2 has an opening 63 at a side surface thereof. The light-emitting element 61 and the light-receiving element 62 are directed to the outside through the opening 63.

An annular shoulder portion 64 is formed in the opening 63 in such a manner that it protrudes inwardly. A glass plate 66 is mounted on the shoulder portion 64 through a packing 65 to protect the light-emitting element 61 and the light-receiving element 62. A pair of sensor retaining frames 68 and 69 and a main substrate 70 are gripped between the apparatus body 1 and a rear lid 67. The sensor retaining frames 68 and 69 respectively have grooves 68a and 69a at end portions thereof. The infrared radiation sensor substrate 60 is fitted into these grooves 68a and 69a. Although not shown, various ICs and electric elements are mounted on the infrared radiation sensor substrate 60 and the main substrate 70 in order to energize the light-emitting element 61 and the light-receiving element 62. Further, a screwed pin 71 is inserted into the retaining frame 68 and the main substrate 70, and a screw 72 is threadedly engaged with an internal screw hole formed in the pin 71, whereby a flexible substrate 73 is mounted between a head portion of the screw 72 and the main substrate 70. Accordingly, one end of the flexible substrate 73 is connected to the main substrate 70. The other end of the flexible substrate 73 is soldered to the infrared radiation sensor substrate 60. Consequently, transmission from the main substrate 70 to the light-emitting element 61 and reception from the light-receiving element 62 to the main substrate 70 are enabled. Reference numeral 74 denotes a packing provided between the apparatus body 1 and the rear lid 67.

In the thus-arranged infrared radiation sensor 3, when the operator brings, for example, the bulb of his forefinger into abutment with the glass plate 66 while the sensor 3 is energized, the light emitted by the light-emitting element 61 is reflected by the forefinger. The reflected light is detected by the light-receiving element 62. At that time, maxima and minima of the reflected light represent the pulsating flow of a blood, thereby enabling measurement of the pulse rate.

Referring to Fig. 1, reference numeral 4 denotes a switch (operation switch) for starting and stopping the measurement by the infrared radiation sensor 3. When the switch is depressed, the light-emitting element 61 and the light-receiving element 62 are turned on or off. Reference numeral 5 denotes a liquid crystal panel for displaying the pulse rate obtained on the basis of the results of the measurement of the infrared radiation sensor 3.

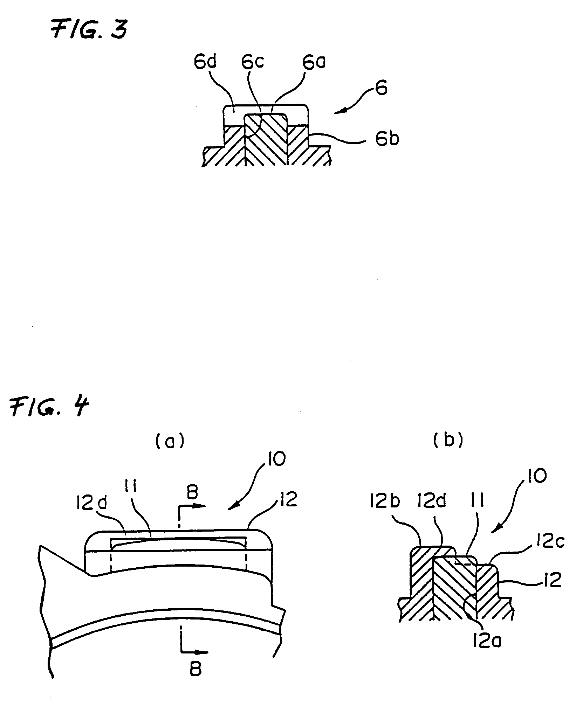

An operation button 6 is mounted on the side portion of the apparatus body 1 at a position opposite to that of the infrared radiation sensor 3. When the operator depresses the operation button 6, the results of the measurement of the infrared radiation sensor 3 are stored in a memory incorporated in the apparatus body 1. As shown in Fig. 3, the operation button 6 includes a button body 6a, and a button guide 6b for slidably supporting the button body 6a in a direction in which the button body 6a is operated. The button body 6a is accommodated in a through-hole 6c formed in the button guide 6b, and is urged in an outward direction by a spring which is not shown. An end surface of the button guide 6b has a convex curved surface 6d curved along a direction perpendicular to the surface of Fig. 3. The outermost edge portion of the convex curved surface 6d protrudes from an end surface of the button body 6a. The end surface of the button body 6a protrudes from the central and innermost portion of the convex curved surface 6d.

Further, the apparatus body 1 includes a sensor for measuring the atmospheric temperature, the atmospheric pressure, the humidity, the intensity of ultraviolet radiation, the wind speed or the frequency (e.g., the stroke of jogging) from the acceleration generated from the movement of the user, and a clock or a stop watch. Reference numerals 7 denote switches (operation buttons) for starting and stopping the measurement of the sensor or the like (for instance a stop watch function) and for selecting the measurement item from the above-mentioned ones. Reference numeral 8a denotes a liquid crystal panel for displaying the results of the measurement of the clock or the stop watch. Reference numeral 8b denotes a liquid crystal panel for displaying the results of the measurement of, for example, the atmospheric temperature or the atmospheric pressure. Reference numeral 8c denotes a liquid crystal panel for displaying the results of the measurement of the pulse rate or the frequency. The items whose measurement results are displayed by the liquid crystal panels 8a, 8b and 8c can be varied by depressing the switches 7. In this embodiment, only the operation button 6 has such a special structure as that described above. However, the switch 4 or the switches 7 may have a similar structure.

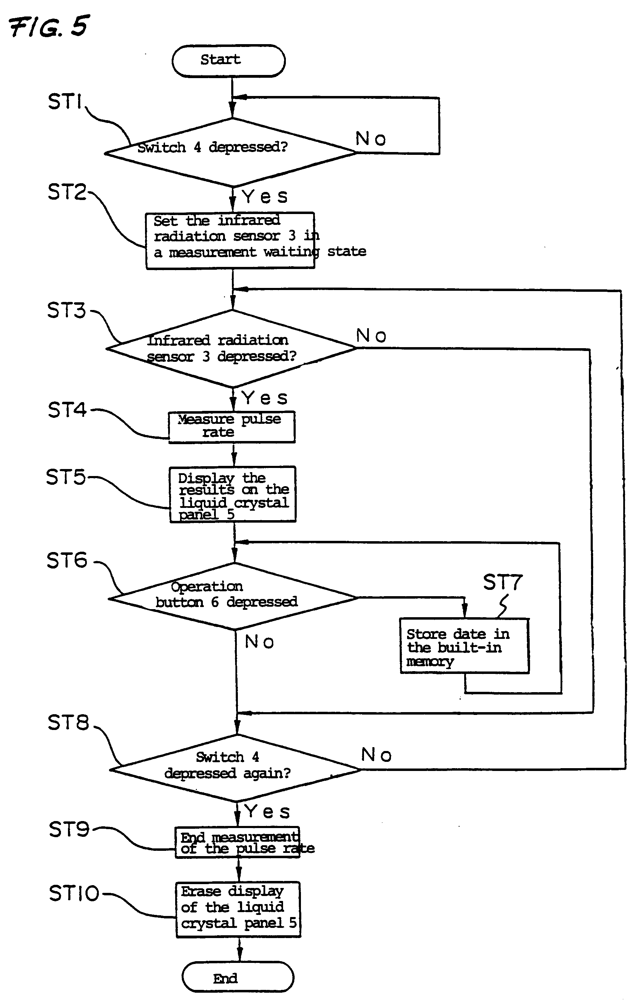

The procedures of measuring the pulse rate in the above-described portable apparatus will now be described with reference to Fig. 5. First, the user depresses the switch 4 to set the infrared radiation sensor 3 in a waiting state in which measurement is enabled (ST1 to ST2). Next, the user grips the infrared radiation sensor 3 and the operation button 6 with his or her forefinger and thumb, respectively, whereby the forefinger is pressed against the infrared radiation sensor 3 and the measurement of the pulse rate of the forefinger is started (ST3 to ST4). While the finger is pressed against the infrared radiation sensor 3, measurement of the pulse rate continues, and the results of the measurement of the pulse rate is transmitted to and displayed by the liquid crystal panel 5 (ST5). At that time, the user's thumb rests on the protruding edge portion of the button guide 6b, and does not press the button body 6a strongly.

Next, if the user desires to memorize the pulse rate, he or she slightly slides the thumb sideways or twists it and depresses the button body 6a with the bulb of his or her thumb to push down the button body 6a against the urging force of the spring (ST6), whereby the results of the measurement of the infrared radiation sensor 3 is input in the built-in memory (ST7). Input of the results of the measurement is stopped by weakening the pressing force on the button body 6a.

When the user depresses the switch 4 again, the measurement of the pulse rate by the infrared radiation sensor 3 is completed, and display of the liquid crystal panel 5 turns off (ST8 to ST10).

To operate the above-described portable apparatus, the user first depresses the switch 4 with, for example, his or her forefinger, and then presses his or her forefinger against the infrared radiation sensor 3. Thereafter, the user depresses the operation button 6 strongly at an appropriate time after pressing his or her forefinger against the infrared radiation sensor 3 while still pressing against the infrared radiation sensor 3. The user continues pressing against the infrared radiation sensor 3 and the operation button 6 simultaneously with his or her forefinger and thumb, respectively. Thereafter, the user depresses the switch 4 again. Here, since the infrared radiation sensor 3 and the operation button 6 are disposed on opposite sides of the apparatus body 1, the user can perform series of operations, such as measurement and recording of the pulse rate, while firmly holding the apparatus body 1 with his or her fingers, thus performing stable measurement. Further, since the infrared radiation sensor 3, the operation button 6 and the switches 4 and 7 are provided on the side portion of the apparatus body 1, the user can move his or her fingers naturally, enabling operability to be greatly improved.

Fig. 4 shows a modification of the operation button in this embodiment. As in the case of the operation button 6, an operation button 10 shown in Fig. 4 includes a button body 11 and a button guide 12. The button guide 12 has a through-hole 12a in which the button body 11 is accommodated. An end surface of the button guide 12 has an upper shoulder portion 12b located at a position of the thumb's nail, and a lower shoulder portion 12c which is lower than the upper shoulder portion 12b. A hanging portion 12d, serving as a stopper of the button body 11, is formed in the upper shoulder portion 12b in such a manner that it hangs halfway over an opening of the through-hole 12a.

In the physiological parameter data measuring apparatus having the above-described operation button 10, the user measures the pulse rate while pressing the hanging portion 12d of the button guide 12 with the bulb of his or her thumb. To record the results of the measurement, the user slightly tilts the thumb and depresses the end surface of the button body 11 with the bulb of his or her thumb. In this modification, since the button guide 12 has the hanging portion 12d, the user can firmly hold the infrared radiation sensor and the operation button 10, and malfunction of the infrared radiation sensor or the operation button can thus be prevented. As in the case of the operation button 6 shown in Fig. 1, the structure of the operation button 10 can also be applied to the switch 4 or 7.

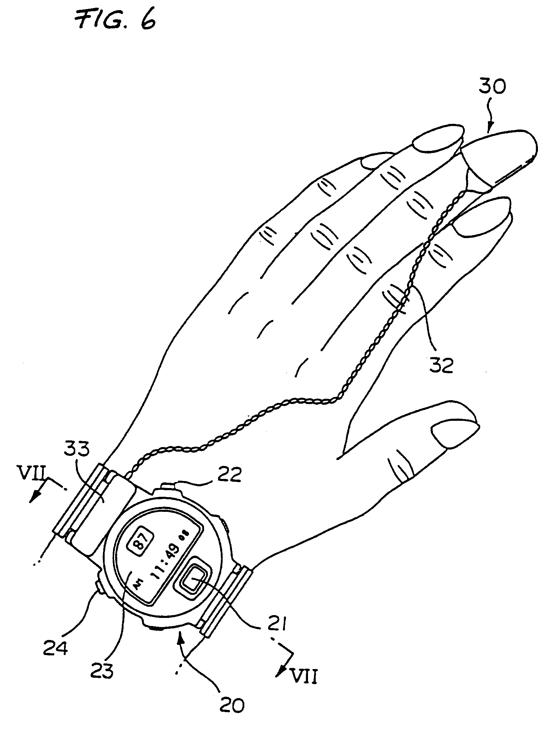

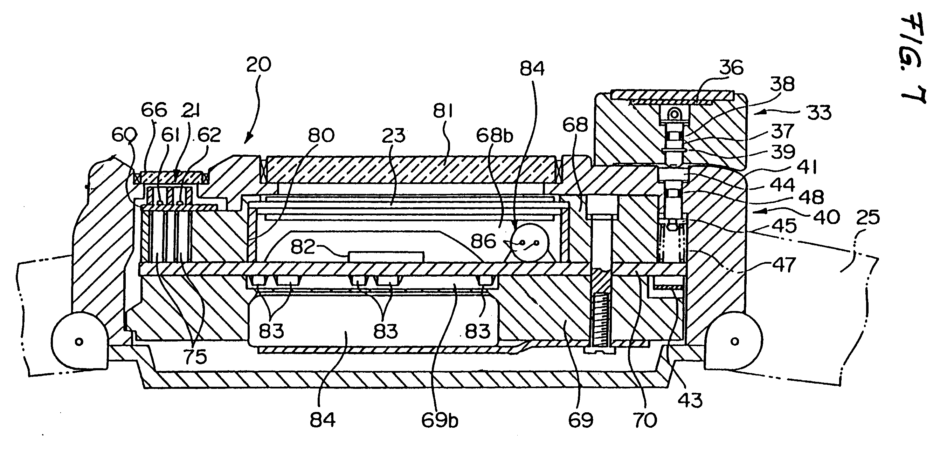

An embodiment of the present invention will be described below with reference to Figs. 6 through 11. The portable apparatus shown in these figures includes a wrist side measuring device 20, and an external measuring device 30 which can be detachably mounted on the wrist side measuring device 20. The wrist side measuring device 20 has an infrared radiation sensor 21 for measuring the pulse rate on a front surface thereof, and a switch 22 used to start and stop the measurement by the infrared radiation sensor 21 on a side thereof. Reference numeral 23 denotes a liquid crystal panel for displaying the results of the measurement. As shown in Fig. 7, the wrist side measuring device 20 has a three-layer internal structure consisting of upper and lower retaining frames 68 and 69 and a main substrate 70 disposed therebetween. The infrared radiation sensor 21 includes an infrared radiation sensor substrate 60 fixed to the upper retaining frame 68, and a light-emitting element 61 and a light-receiving element 62 connected to the infrared radiation sensor substrate 60. A glass plate 66 is mounted on the surface of the wrist side measuring device 20 to protect the light-emitting element 61 and the light-receiving element 62. The light-emitting element 61 and the light-receiving element 62 are electrically connected to the main substrate 70 through coil springs 75.

The retaining frame 68 has an opening 80 in a center thereof. The liquid crystal panel 23 is accommodated in the opening 80. A glass plate 81 is mounted on the surface of the wrist side measuring device 20 to protect the liquid crystal panel 23. Various ICs 82 and electric elements 83 mounted on the main substrate 70 are accommodated in internal spaces 68b and 69b provided in the retaining frames 68 and 69, respectively. In this embodiment, sensors for measuring the atmospheric temperature, the atmospheric pressure, the humidity, the intensity of ultraviolet radiation, the wind speed or the frequency from the acceleration generated by the movement of the user may also be selectively built in the apparatus, as in the case of the first embodiment. The results of the measurement by any of such sensors are displayed by the liquid crystal panel 23. In Fig. 7, an acceleration sensor 84 for measuring a stride of the arm of the user who is doing an exercise, such as jogging is shown.

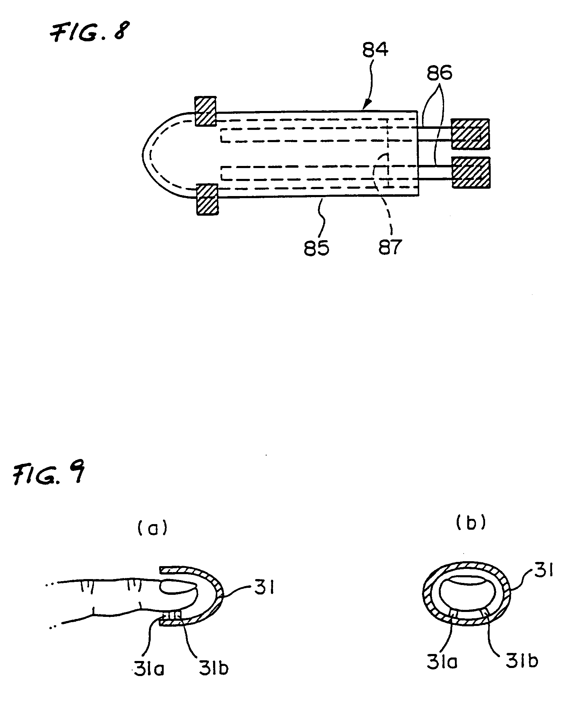

As shown in Fig. 8, the acceleration sensor 84 is constructed such that two bar-like terminals 86 fixed to a single piezoelectric element (not shown) are inserted into a quartz tube 85 having a diameter of 2 mm and a length of 8 mm. Reference numeral 87 denotes a lid for closing the quartz tube 85 with the terminals 86 inserted therein. The two ends of the acceleration sensor 84, i.e., the quartz tube 85 and the terminals 86, are soldered to the main substrate 70, whereby the piezoelectric element is deformed by an acceleration applied in a direction (which is parallel to the substrate 70) perpendicular to the longitudinal direction of the quartz tube 85. Deformation of the piezoelectric element is detected by the ICs 82 on the main substrate 70, and an acceleration value is displayed by the liquid crystal panel 23 in response to a detection signal.

Thus, the acceleration sensor 84 is disposed such that the direction in which the acceleration is generated, i.e., in which the user swings his or her arm and the longitudinal direction of the quartz tube 85 are perpendicular to each other, as shown in Fig. 7. However, the position at which the acceleration sensor 84 is provided is not limited, if it does not interfere with the liquid crystal panel 23, the ICs 82 and the electric element 83 and if it allows the terminals 86 to be connected to the main substrate 70. The form of the acceleration sensor is not limited to that shown in this embodiment and the acceleration sensor can have any other forms.

In the measurement of the pulse rate of the user who is doing exercise, since the movement of the physical body of the user affects measurement of the pulse rate in the form of noises, the pulse rate measured by the infrared radiation sensor 21 may differ from the true pulse rate. Hence, in this embodiment, the true pulse rate is calculated from the pulse rate obtained by the infrared radiation sensor 21 and the acceleration detected by the acceleration sensor 84 in a circuit on the main substrate 70 by the method disclosed in, for example, JP-A-60-135029. The infrared radiation sensor 21 measures the pulse rate of the finger of the arm different from that on which the wrist side measuring device 20 is mounted. However, the infrared radiation sensor 21 is pressed by the finger during measurement, making the accelerations of the two arms equal to each other. Consequently, it is possible in this embodiment to remove the noises generated by the movement of the user's body and thereby display an accurate pulse rate on the liquid crystal panel 23.

The acceleration sensor 84 may also be provided in the embodiments shown in Figs. 1 through 5. In that case, an accurate pulse rate can be obtained by the same method as that employed in this embodiment.

As shown in Fig. 9, the external measuring device 30 includes a finger stall-like infrared radiation sensor 31 in which a light-emitting element 31a and a light-receiving element 31b protrude inwardly, and a connector 33 connected to the infrared radiation sensor 31 via a lead 32. The external measuring device 30 measures the pulse rate, like the wrist side measuring device 20, and starts or stops measurement when the user depresses a switch 24 disposed on the wrist side measuring device 20. In addition to the infrared radiation sensor for measuring the pulse rate, the external measuring device may also incorporate a sensor for measuring the blood pressure, the body temperature or the heart beat. Further, the form of the external measuring device is not limited to that mounted on the fingertip. The external measuring device may also be formed such that it can be mounted on the root of the finger using a magic tape, may be shaped in a clip-like form so that it can be held on an ear or may be shaped in a glove-like form.

Where the external measuring device 30 is designed to measure the pulse rate, the true pulse rate may be calculated in the circuit on the main substrate 70 from both the measured pulse rate and the acceleration detected by the acceleration sensor 84. No acceleration sensor is provided on the finger side, because the acceleration sensor has too big a size to be mounted on the finger, because there is the possibility that the quartz tube breaks when something strikes the acceleration sensor mounted on the finger and mainly because it detects the fine motion of the finger when mounted on the finger, thus disabling accurate measurement of the stride of the arm. For the case of measurement of the pulse rate, since an ordinary user may not move the finger very much during exercise, the pulse rate is hardly affected by the acceleration generated by the movement of the finger. It is therefore estimated that an accurate pulse rate can be obtained using the acceleration generated by the stride of the arm.

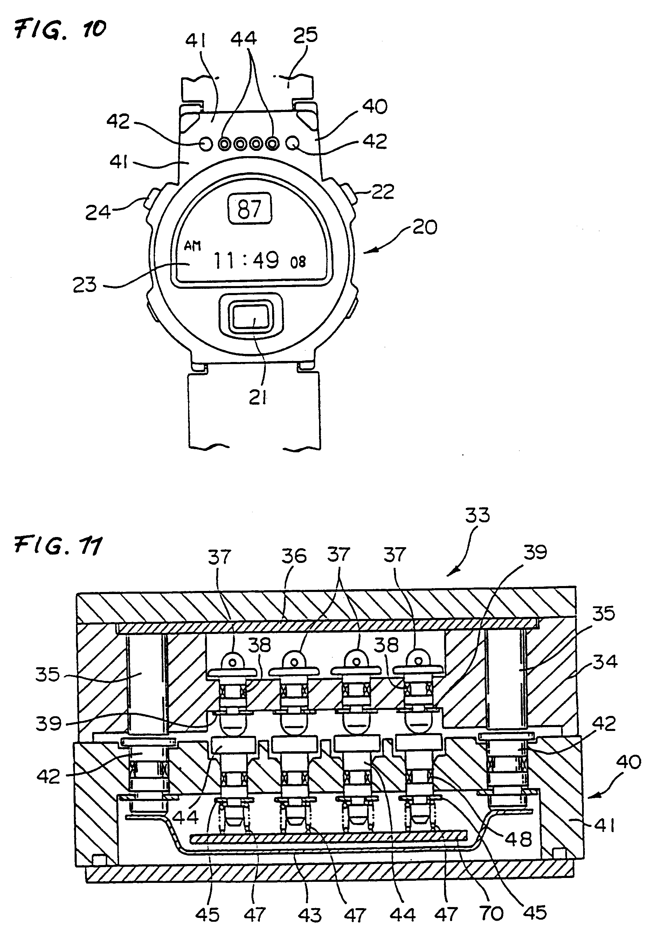

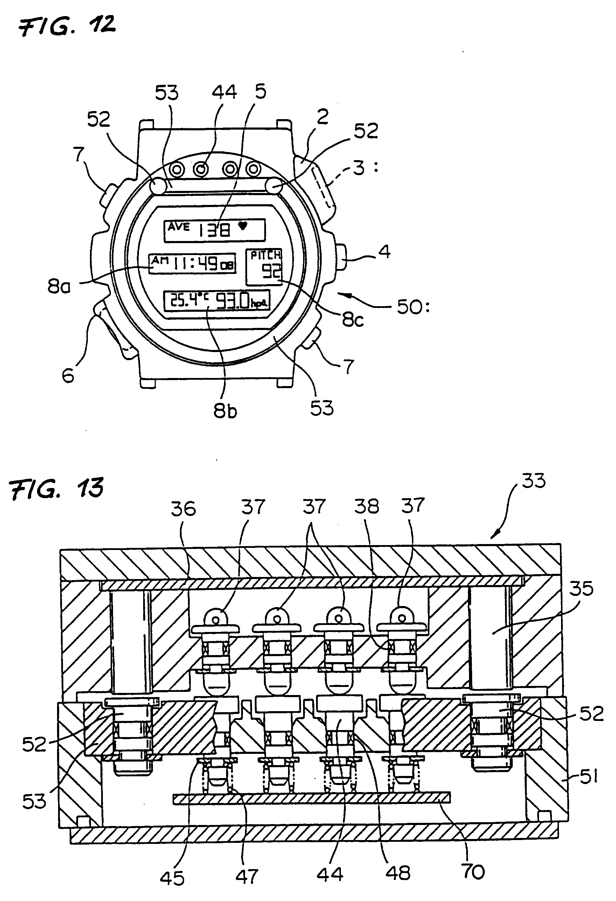

As shown in Figs. 10 and 11, the connector 33 of the external measuring device 30 and the connector 40 of the wrist side measuring device 20 which receives the connector 33 have the following structures: in a connector body 34, permanent magnets 35 are disposed in such a manner that they are separated from each other. One end of each of the permanent magnets 35 protrudes from an undersurface of the connector body 34. The other end surfaces of the permanent magnets 35 are connected with each other by a plate member 36 made of a soft magnetic material. At the central portion of the connector body 34, terminals 37 are mounted in such a manner that axes thereof are directed in the vertical direction. In the figure, reference numeral 38 denotes a water-proof packing. Reference numeral 39 denotes a washer for preventing coming off of the terminal..

A connector body 41 of the wrist side measuring device 20 has a form which allows it to extend toward a band 25 (see Fig. 10). In the connector body 41, magnetizable members 42 made of a soft magnetic material are mounted in such a manner that they face the permanent magnets 35 when the connector 33 is connected to the connector 40. An upper end of each of the magnetizable members 42 protrudes from an upper surface of the connector body 41. The lower end surfaces of the magnetizable members 42 are connected to each other by a plate member 43 made of a soft magnetic material. At the central portion of the connector body 41, terminals 44 are mounted in such a manner that axes thereof are directed in the vertical direction and that they are movable in the vertical direction by a predetermined distance. A washer 45 is mounted on the lower end portion of each of the terminals 44. The connector body 41 also incorporates the portion of the main substrate 70 so that a coil spring 47 can be mounted between the main substrate 70 and the washer 45 to push up the terminal 44. This structure of the connector 40 allows the terminals 44 to be pressed against the end surfaces of the terminals 37 of the external measuring device 30 when the connector 33 is connected to the connector 40. Reference numeral 48 denotes a water-proof packing.

In the thus-arranged portable apparatus, it is possible to continuously measure the pulse rate while the external measuring device 30 is connected to the wrist side measuring device 20. Even if the user has forgotten to take the external measuring device 30, it is possible to obtain not only the pulse rate from the wrist side measuring device 20 but also physiological parameter data which can be an important index of the condition of one's health, such as the blood pressure or the body temperature, and environment data, such as the humidity or the atmospheric temperature. Consequently, the above-described portable apparatus enables the user to carry out health care during exercise reliably, thus preventing any accident which would happen during exercise.

The stride of the user's arm when the user is, for example, jogging, can be obtained by the acceleration sensor 84. The data obtained by the acceleration sensor 84 can also be used to remove an influence of the acceleration generated by the movement of the user's body from the pulse rate measured by the infrared radiation sensor 21 and thereby calculate and display the true pulse rate on the liquid crystal panel 23.

Furthermore, in the above-described portable apparatus, when the external measuring device 30 is mounted on the wrist side measuring device 20, a loop of lines of magnetic force is formed through the permanent magnets 35, the magnetizable members 42 and the plate members 36 and 43 made of a soft magnetic material. Consequently, a sufficient magnetic force can be obtained even when no magnet is provided on the wrist side measuring device 20, and the connectors 33 and 40 can thus be firmly connected to each other. Furthermore, since no magnet is provided in the wrist side measuring device 20, problems involving attachment of dust, such as iron sand, and erasure of the data on the magnetic card the user possesses, can be prevented.

Furthermore, since the connector 33 of the external measuring device 30 is constructed so that it is connected to the front surface portion of the wrist side measuring device 20, the pressing force the user applies to the wrist side measuring device 20 when he or she mounts the external measuring device 30 is supported by the user's arm, thus enabling smooth mounting of the external measuring device 30. Further, since the connector 33 of the external measuring device 30 does not protrude from the side of the wrist side measuring device 20, an uncomfortable feeling, such as the protruding portion of the connector 33 touching the back of the hand, can be eliminated.

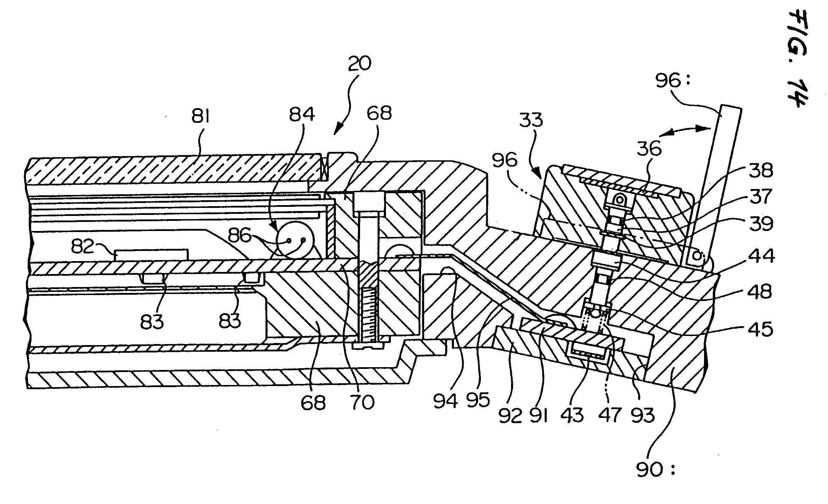

Fig. 14 illustrates a modification of the connector 40. In Fig. 14, the connector 40 is not provided on the wrist side measuring device 20 (unlike the case shown in Fig. 7) but provided on the surface of a band 90. The band 90 is formed integrally with the wrist side measuring device 20. The connector 40 has the same structure as that described above with the exception that it is supported not by the main substrate 70 but by a connector substrate 91. In this modification, the same connector 33 as that described above is used.

A space 92 for accommodating the connector substrate 91 is formed on the rear side of the band 90. The connector substrate 91 is disposed in the space 93 in a state wherein it is mounted on a mounting plate 92. Also, a cavity 94 for communicating the interior of the wrist side measuring device 20 with the space 93 is formed in the band 90, and a flexible substrate 95 is inserted into the cavity 94 so as to connect the connector substrate 91 to the main substrate 70, whereby transmission and reception between the main substrate 70 and the connector 40 is enabled. Further, a cover 96 is provided pivotally on the front of the band 90. The cover 96 is urged toward the band 90 by a spring which is not shown. To connect the connectors 33 and 40, the cover 96 is raised against the urging force of the spring. When the external measuring device 30 is not used, i.e., when the connectors 33 and 40 are not connected, the connector 40 is closed by the cover 96.

In this modification, since the connector 40 is provided on the band 90, the size of the wrist side measuring device 20 can be further reduced. Accordingly, a gap formed between the wrist side measuring device 20 and the user's wrist can be reduced, and consequently, the texture with which the user wears the measuring device 20 can be improved. Further, shift of the measuring device 20 along or about the wrist, caused by the user's movement, can be eliminated. Further, since the connector 40 is closed by the cover 96 urged by the spring when the external measuring device 30 is not used, the external view of the measuring device 20 is improved while entrance of water droplets from outside can be prevented, thus preventing the user's receiving an electric shock. Closing of the connector 40 with the cover 96 also enables the connector 40 and hence, the electric parts in the wrist side measuring device to be protected from an externally applied static electricity. The structure of the cover 96 is not limited to the above-described one and the cover 96 may also be provided slidably.

Figs. 12 and 13 show another embodiment of the present invention. The portable measuring apparatus shown in Figs. 12 and 13 differs from the portable apparatus shown in Figs. 1 through 11 and 14 in that magnetizable members of a wrist side measuring device 50 are provided along a peripheral edge portion of a front surface thereof. Reference numerals in these figures identical to those in Figs. 1 through 11 and 14 represent similar or identical elements, description thereof being omitted. In a connector body 51, a coupling member 53 for coupling magnetizable members 52 in a straight line and for extending along the peripheral edge portion of the front surface of the wrist side measuring device 50 in an arc form is mounted. The coupling member 53 is made of a soft magnetic material. As a result, when the external measuring device is mounted, a loop of lines of magnetic force which circles along the peripheral edge portion of the front surface of the wrist side measuring device 50 is formed. The connector body 51 is provided on the front surface of the wrist side measuring device 50. However, the connector body 51 may also be provided on the front surface of the band (not shown), as in the case of the above-described modification.

In the thus-arranged portable apparatus, since the loop of lines of magnetic force is formed on the front surface portion of either the wrist side measuring device 50 or the band, an influence of the electric noises caused by the static electricity generated by the lines of magnetic force on the electric circuits of the wrist side measuring device 50 can be reduced. Further, exposure of the coupling member 53 on the front surface of the wrist side measuring device 50 can be utilized as a design.

The present invention is characterized in the connection structure for connecting the wrist side apparatus body or the band used to wind the apparatus body around the arm to the external unit in which, for example, the external measuring device is incorporated.

Furthermore, in the present invention, since a loop of lines of magnetic force is formed in at least either the magnets of the connector of the external unit or the magnetizable members of the wrist side apparatus body, a sufficient magnetic force can be obtained and the connector can be firmly connected even when no magnet is provided in the apparatus body. Further, since no magnet is provided in the apparatus body, problems involving attachment of dust, such as iron sand, or erasure of the data on a magnetic card can be prevented.

## Claims

1. A portable measuring apparatus for measuring one or more physiological parameters of a user, comprising
a wrist watch type apparatus body (1), a band (90) the user winds around his or her arm,
a wrist side measuring device (20) disposed on said wrist watch type apparatus body and having a measuring unit (3), serving as a physiological parameter sensor; and
an external measuring device (30, 32) detachably connected to said wrist side measuring device and adapted to measure physiological parameter data,
**characterized in that** said wrist side measuring device (20) incorporates an environment data measuring mechanism for measuring environment data and has a connector (40; 50) for detachably connecting said external measuring device (30, 32) to said wrist side measuring device, said connector being provided on a front surface portion of either said apparatus body (1) or of said band (90), wherein magnetic means are provided whose magnetic force is utilized to connect said apparatus body (1) and said external measuring device (30, 32) to each other, said magnetic means having
at least two magnets (35) which are disposed in a matching connector (33) of said external device (30, 32) in such a manner that said at least two magnets are separated from each other, and
magnetizable members (42; 52) made of a soft magnetic material which are disposed in either said apparatus body (1) or said band in such a manner that said magnetizable members face said magnets,
wherein said magnetizable members and/or said magnets are connected to each other
by a soft magnetic material (43; 53; 36).

2. The apparatus according to claim 1, wherein said environment data measuring mechanism has an acceleration measuring unit (84) for measuring an acceleration generated by a user's motion to offer a frequency thereof.

3. The apparatus according to claim 2, further comprising means (70) for correcting said physiological parameter data by the accelereation measured by said acceleration measuring unit (84).

4. The apparatus according to claim 1 or 2, wherein said connector (40) is provided on the front surface of said band (90), said band having a movable cover (96) to cover said connector.

5. The apparatus according to claim 1 or 2, wherein said magnetizable members are connected by said soft magnetic material (53) being arranged to circle along a peripheral edge portion of a front surface of said apparatus body (1).

6. The apparatus according to any one of the preceding claims, wherein said measuring unit (3) is disposed at a first position on a side portion of said apparatus body (1), and further comprising
at least one operation button (6; 10) for operating electric parts provided in said apparatus body (1), said operation button (6; 10) being disposed at a second position on said side portion different from said first position,
said apparatus being adapted, for performing measurement by means of said measuring unit (3), to be firmly gripped between two fingers of a user's hand, one finger at said first position and another at a third position on said side surface substantially diametrically opposite to said first position,
wherein said second position and said third position are the same such that the user is enabled to perform measurement with one finger pressed against said measuring unit (3) while at the same time the other finger is capable of operating said operation button (6; 10).

7. The apparatus according claim 6, wherein said operation button (6; 10) includes a button body (6a; 11), and a button guide (6b; 12) for moveably supporting said button body (6a; 11) in a direction in which said button body (6a; 11) is operated, said button guide (6b; 12) having a support surface protruding from an operation surface of said button body (6a; 11).

## Patentansprüche

1. Tragbares Meßgerät zum Messen eines oder mehrerer physiologischer Parameter eines Benutzers oder einer Benutzerin, umfassend
ein armbanduhrartiges Gerätegehäuse (1),
ein Band (90), das der Benutzer oder die Benutzerin um seinen bzw. ihren Arm schlingt,
eine handgelenkseitige Meßvorrichtung (20), die an dem armbanduhrartigen Gerätegehäuse angeordnet ist und eine Meßeinheit (3) aufweist sowie als Sensor für physiologische Parameter dient; und
eine externe Meßvorrichtung (30, 32), die mit der handgelenkseitigen Meßvorrichtung lösbar verbunden und ausgebildet ist, um Daten physiologischer Parameter zu messen,
**dadurch gekennzeichnet**, daß die handgelenkseitige Meßvorrichtung (20) einen Umgebungsdatenmeßmechanismus zum Messen von Umgebungsdaten umfaßt und ein Steckverbinderteil (40; 50) zum lösbaren Verbinden der externen Meßvorrichtung (30, 32) mit der handgelenkseitigen Meßvorrichtung aufweist, wobei das Steckverbinderteil an einem Vorderseitenabschnitt entweder des Gerätegehäuses (1) oder des Bandes (90) vorgesehen ist, wobei eine Magnetanordnung vorgesehen ist, deren Magnetkraft dazu verwendet wird, das Gerätegehäuse (1) und die externe Meßvorrichtung (30, 32) miteinander zu verbinden, wobei die Magnetanordnung aufweist
zumindest zwei Magnete (35), die in einem passenden Steckverbinderteil (33) der externen Vorrichtung (30, 32) so angeordnet sind, daß die zumindest zwei Magnete voneinander getrennt sind, und
magnetisierbare Elemente (42; 52) aus weichmagnetischem Material, die entweder in dem Gerätegehäuse (1) oder dem Band so angeordnet sind, daß die magnetisierbaren Elemente den Magneten zugewandt sind,
wobei die magnetisierbaren Elemente und/oder die Magnete durch ein weichmagnetisches Material (43; 53; 36) miteinander verbunden sind.

2. Gerät nach Anspruch 1, bei dem der Umgebungsdatenmeßmechanismus eine Beschleunigungsmeßeinheit (84) zum Messen einer Beschleunigung aufweist, die durch die Bewegung eines Benutzers oder einer Benutzerin erzeugt wird, um deren Frequenz zu liefern.

3. Gerät nach Anspruch 2, ferner umfassend eine Anordnung (70) zum Korrigieren der Daten physiologischer Parameter durch die von der Beschleunigungsmeßeinheit (84) gemessene Beschleunigung.

4. Gerät nach Anspruch 1 oder 2, bei dem das Steckverbinderteil (40) auf der Vorderfläche des Bandes (90) vorgesehen ist, wobei das Band einen beweglichen Deckel (96) aufweist, um das Steckverbinderteil abzudecken.

5. Gerät nach Anspruch 1 oder 2, bei dem die magnetisierbaren Elemente durch das weichmagnetische Material (53) verbunden sind, das so angeordnet ist, das es längs eines Umfangsrandabschnitts einer Vorderfläche des Gerätegehäuses (1) umläuft.

6. Gerät nach einem der vorhergehenden Ansprüche, bei dem die Meßeinheit (3) an einer ersten Position an einem Seitenabschnitt des Gerätegehäuses (1) angeordnet ist, und das ferner umfaßt
zumindest einen Bedienungsknopf (6; 10) zum Bedienen elektrischer Teile, die in dem Gerätegehäuse (1) vorgesehen sind, wobei der Bedienungsknopf (6; 10) an einer von der ersten Position verschiedenen zweiten Position an dem Seitenabschnitt angeordnet ist,
wobei das Gerät zum Ausführen einer Messung mittels der Meßeinheit (3) so ausgebildet ist, daß es zwischen zwei Fingern der Hand eines Benutzers oder einer Benutzerin fest gegriffen werden kann, ein Finger an der ersten Position und ein anderer an einer dritten Position an der Seitenfläche, die der ersten Position im wesentlichen diametral gegenüberliegt,
wobei die zweite Position und die dritte Position gleich sind, so daß der Benutzer oder die Benutzerin eine Messung mit einem gegen die Meßeinheit (3) gedrückten Finger ausführen kann, während der andere Finger gleichzeitig den Bedienungsknopf (6; 10) bedienen kann.

7. Gerät nach Anspruch 6, bei dem der Bedienungsknopf (6; 10) einen Knopfkörper (6a; 11) und eine Knopfführung (6b; 12) zur beweglichen Halterung des Knopfkörpers (6a; 11) in einer Richtung aufweist, in welcher der Knopfkörper (6a; 11) betätigt wird, wobei die Knopfführung (6b; 12) eine Haltefläche aufweist, die von einer Bedienungsfläche des Knopfkörpers (6a; 11) hervorragt.

## Revendications

1. Dispositif de mesure portatif destiné à mesure un ou plusieurs paramètres physiologiques d'un utilisateur, comportant :
un corps (1) de dispositif de type montre de poignet,
une bande (90) que l'utilisateur enroule autour de son bras,
un dispositif (20) de mesure latérale de poignet disposé sur le corps de dispositif de type montre de poignet et ayant une unité (3) de mesure, servant en tant qu'un capteur de paramètre physiologique ; et
un dispositif (30, 32) de mesure extérieure connecté de manière amovible au dispositif de mesure latérale de poignet et conçu pour mesurer des données de paramètre physiologique,
caractérisé en ce que le dispositif (20) de mesure latérale de poignet incorpore un mécanisme de mesure de données d'environnement destiné à mesurer des données liées à l'environnement et comporte un connecteur (40 ; 50) destiné à relier de manière amovible le dispositif (30, 32) de mesure extérieure au dispositif de mesure latérale de poignet, le connecteur étant-disposé sur une partie de surface avant de soit le corps (1) de dispositif, soit de la bande (90), dans lequel des moyens magnétiques sont prévus dont la force magnétique est utilisée pour relier le corps (1) de dispositif et le dispositif (30, 32) de mesure extérieure mutuellement, les moyens magnétiques ayant
au moins deux aimants (35) qui sont disposés dans un connecteur (33) correspondant du dispositif (30, 32) extérieur d'une manière telle que lesdits au moins deux aimants sont séparés l'un de l'autre, et
des éléments (42 ; 52) pouvant être magnétisés réalisés à partir d'un matériau magnétique doux qui sont disposés soit dans le corps (1) du dispositif soit dans la bande d'une manière telle que les éléments pouvant être magnétisés font face aux aimants,
dans lequel les éléments pouvant être magnétisés et/ou les aimants sont reliés mutuellement par un matériau (43, 53, 36) magnétique doux.

2. Dispositif suivant la revendication 1, dans lequel le mécanisme de mesure de données liées à l'environnement comporte une unité (84) de mesure d'accélération destinée à mesurer une accélération produite par un déplacement de l'utilisateur pour obtenir une fréquence de celui-ci.

3. Dispositif suivant la revendication 2, comportant en outre des moyens (70) destinés à corriger les données de paramètre physiologique par l'accélération mesurée par l'unité (84) de mesure d'accélération.

4. Dispositif suivant la revendication 1 ou 2, dans lequel le connecteur (40) est disposé sur la surface avant de la bande (90), la bande ayant un capot (96) mobile pour couvrir le connecteur.

5. Dispositif suivant la revendication 1 ou 2, dans lequel les éléments pouvant être magnétisés sont reliés par le matériau (53) magnétique doux qui est agencé de manière à former un cercle le long d'une partie de bord périphérique d'une surface avant du corps (1) de dispositif.

6. Dispositif suivant l'une quelconque des revendications précédentes, dans lequel l'unité (3) de mesure est disposée à une première position sur une partie latérale du corps (1) de dispositif, et comportant en outre
au moins un bouton (6 ; 10) de fonctionnement destiné à faire fonctionner des parties électriques disposées dans le corps (1) de dispositif, le bouton (6 ; 10) de fonctionnement étant disposé à une seconde position sur la partie latérale différente de la première position,
le dispositif étant conçu, pour effectuer des mesures au moyen de l'unité (3) de mesure, pour être serré de manière ferme entre deux doigts d'une main d'utilisateur, un doigt à la première position et un autre à une troisième position sur la surface latérale sensiblement diamétralement opposée à la première position,
dans lequel la seconde position et la troisième position sont la même de sorte que l'utilisateur peut effectuer des mesures avec un doigt pressé contre l'unité (3) de mesure tandis que simultanément l'autre doigt est capable de faire fonctionner le bouton (6 ; 10) de fonctionnement.

7. Dispositif suivant la revendication 6, dans lequel le bouton (6 ; 10) de fonctionnement inclut un corps (6a ; 11) de bouton et un guide (6b ; 12) de bouton pour supporter de manière amovible le corps (6a ; 11) de bouton dans une direction dans laquelle le corps (6a ; 11) de bouton est mis en fonctionnement, le guide (6b ; 12) de bouton ayant une surface de support faisant saillie d'une surface de fonctionnement du corps (6a ; 11) de bouton.
